# EUROPEAN PATENT APPLICATION

(11) **EP 2 790 024 A1**
(43) Date of publication of application: **15.10.2014**
(21) Application number: 14162164.9
(22) Date of filing: 28.03.2014
(51) Int. Cl.: G01N 33/86

(54) **Method and reagent kit for measuring activated partial thromboplastin time**

(30) Priority: 28.03.2013 JP 2013069367
(71) Applicant: SYSMEX CORPORATION, Kobe-shi, Hyogo 651-0073 (JP)
(72) Inventor: Okuda, Masahiro, Kobe-shi, Hyogo 651-0073 (JP); Kinoshita, Emi, Kobe-shi, Hyogo 651-0073 (JP); Suzuki, Takeshi, Kobe-shi, Hyogo 651-0073 (JP)
(74) Representative: Paemen, Liesbet R.J.

(57) **Abstract**

The present invention provides a method for measuring activated partial thromboplastin time. The method comprises: a first mixing step of mixing a blood plasma with a first reagent, wherein the first reagent comprises an activator and phosphatidylglycerol at a concentration equal to or greater than 25 µg/mL; a second mixing step of mixing a sample obtained in the first mixing step with a second reagent comprising a calcium salt; and a step of measuring coagulation time of the sample obtained in the second mixing step.

## Description

### FIELD OF THE INVENTION

The present invention relates to a method for measuring activated partial thromboplastin time (APTT), and a reagent kit for APTT measurement.

### BACKGROUND

In the field of clinical tests, there are known tests wherein blood coagulation time is measured to check, for example, abnormalities of blood coagulation factors. One of the conventional clinical tests performed to check blood coagulability is activated partial thromboplastin time (APTT) measurement. The APTT is a coagulation time which reflects functions of intrinsic pathways of coagulation initiated by contact of blood with any negatively charged foreign matter during hemorrhage. The APTT measurement is used in the screening of deficiencies or abnormalities of intrinsic coagulation factors and also in monitoring of heparin anticoagulation therapy.

The APTT measurement is a longstanding method used in clinical tests over the years to measure coagulation time. This method uses a negatively charged activator, a phospholipid which is an alternative of platelet factor 3, and a calcium salt to feed calcium ions. The principle of measurement according to this method is described below. To start with, the activator and phospholipid are added to blood plasma so that contact factors in the blood plasma are activated by the activator. When the calcium salt is further added to the blood plasma, actions of the activated contact factors and phospholipid accelerate coagulation, finally depositing fibrin in the blood plasma. The APTT measurement, wherein fibrin deposition is defined as coagulation, counts the time from when the calcium salt is added to when the fibrin is deposited as the coagulation time.

A variety of reagents for APTT measurement have so far been developed. All of these reagents, however, consist primarily of phospholipids. The phospholipids used in the reagents for APTT measurement include naturally-derived phospholipids and synthesized phospholipids. In recent years, synthesized phospholipids of different types have been mixed and used in view of clinical sensitivity, discrimination of disorders, and quality improvement (for example, US Pub. No. 2011/159597). Among the synthesized phospholipids, three different phospholipids; phosphatidylethanolamine (PE), phosphatidylcholine (PC), and phosphatidylserine (PS), are most typically mixed and contained in the reagents.

A known problem in the measurements using such reagents for APTT measurement is the prolonged coagulation time in the cases where Lupus Anticoagulant (LA), an anti-phospholipid antibody, is present in blood samples collected from test subjects, because LA inhibits phospholipids necessary for blood coagulation. Taking advantage of such prolonged coagulation time, LA screening tests are performed these days to detect LA using phospholipid-containing reagents for APTT measurement. These LA screening tests use reagents for APTT measurement containing phospholipids at low concentrations so that the inhibition of phospholipids by LA is more prominent. Some reagent kits available for the detection of LA use a combination of two APTT reagents, each respectively containing phospholipids at different concentrations.

### SUMMARY OF THE INVENTION

The scope of the present invention is defined solely by the appended claims, and is not affected to any degree by the statements within this summary.

In the case where LA is present in blood of a test subject with a suspected disorder associated with a coagulation factor deficiency or abnormality such as coagulopathy or hemophilia, measurements using such conventional reagents for APTT measurement cannot determine whether the prolonged coagulation time results from LA or the coagulation factor deficiency or abnormality. To determine that, another test using a dedicated reagent kit for LA detection or a combination of different tests is conventionally required. Therefore, there is a need for improved APTT measurement which allows the determination of disorders associated with coagulation factor deficiencies or abnormalities.

There have been attempts to lower the sensitivity of the reagents for APTT measurement to LA, such as increasing the phospholipid content of reagents, and increasing the concentrations of hexagonal phosphatidylethanolamine (PE) and negatively charged PS. Yet, so far there have been no reagents for which the sensitivities to LA was effectively weakened or which were proven to be unaffected by LA.

The present invention provides a method for measuring activated partial thromboplastin time (APTT). The method comprises steps of: first mixing a blood plasma with a first reagent, wherein the first reagent comprises an activator and phosphatidylglycerol at a concentration equal to or greater than 25 µg/mL; second, mixing a sample obtained in the first mixing step with a second reagent comprising a calcium salt; and measuring coagulation time of the sample obtained in the second mixing step.

The present invention further provides a reagent kit for measuring activated partial thromboplastin time. The reagent kit comprises a first reagent comprising phosphatidylglycerol and a second reagent comprising a calcium salt. The concentration of the phosphatidylglycerol in the first reagent is 25 to150 µg/mL.

The present invention further provides a use of the reagent for a method for determining blood coagulation abnormality. The method comprises steps of: first mixing a blood plasma with a first reagent, wherein the first reagent comprises an activator and phosphatidylglycerol at a concentration equal to or greater than 25 µg/mL; second mixing a sample obtained in the first mixing step with a second reagent comprising a calcium salt; measuring coagulation time of the sample obtained in the second mixing step; and determining the presence or absence of a blood coagulation abnormality which is not caused by a lupus anticoagulant in the blood plasma based on the coagulation time obtained in the measuring step.

According to the present invention, the APTT measurement can be performed with less LA-induced impact, and the occurrence of cloudiness and/or precipitation in reagents is prevented.

### DETAILED DESCRIPTION OF THE PREFERRED EMBODIMENTS

The preferred embodiments of the present invention will be described hereinafter with reference to the drawings.

A reagent for APTT measurement (hereinafter, may be simply called "reagent") used in a method according to the present invention contains PG at a concentration equal to or greater than 25 µg/mL. The PG is not particularly limited, and any PG conventionally used in the fields of biology and pharmaceutics may be used. The PG may be any one of synthesized phospholipids and naturally-derived PG obtained from cerebra of animals, tissues of plants, or pellicles of microorganisms. To ensure a good quality of the reagent, however, the PG is preferably a synthesized PG. Though not particularly limited, fatty acid side chains of the PG are, for example, palmitic acid, oleic acid, and stearic acid.

Though not particularly limited, the PG should be contained in the reagent at a concentration equal to or greater than 25 µg/mL. The PG, if contained at a concentration equal to or greater than 200 µg/mL, may not be completely dissolved in the reagent. As a result, the reagent is likely to undergo cloudiness and/or precipitation. To ensure a good quality of the reagent, therefore, the PG concentration preferably stays in such a range of values that avoids the risk of cloudiness and/or precipitation in the reagent. Specifically, the PG concentration is preferably equal to or smaller than 150 µg/mL. According to the preferred embodiment, the PG concentration is equal to or greater than 25 µg/mL. Also, the PG concentration is equal to or smaller than 150 µg/mL to prevent the occurrence of cloudiness and/or precipitation in the reagent at 2 to 8°C. A particularly preferable example of the PG concentration is 50 to 150 µg/mL.

A solvent used in the reagent is not particularly limited as far as PG can be dissolved therein and the APTT measurement is not thereby disturbed. The solvent is preferably a buffer solution conventionally used in reagents for APTT measurement. Examples of the buffer solution are HEPES, TRIS, and MOPS. The pH of the reagent is preferably 5 to 9, and more preferably 6 to 8.

According to the preferred embodiment, the reagent further contains an activator. The activator may be any one of materials that activate contact factors. For example, the activator is at least one selected from ellagic acid, kaolin, cerite, colloidal silica, and silicic anhydride. Of these examples, ellagic acid or colloidal silica is preferably used.

The concentration of the activator included in the reagent is not particularly limited. The concentration of the activator is suitably decided depending on the type of the activator and conditions of the APTT measurement. For example, the concentration of the activator is preferably 5 to 1000 µg/mL, and more preferably 20 to 500 µg/mL. When the ellagic acid is used as the activator, the concentration of the ellagic acid included in the reagent is preferably 5 to 100 µg/mL, and more preferably 20 to 50 µg/mL.

According to the preferred embodiment, the reagent further contains a phospholipid other than the PG. The phospholipid other than the PG may be any one of phospholipids conventionally used in the reagent for APTT measurement, for example, at least one selected from PE, PC, and PS. The phospholipid other than the PG may be any one of synthesized phospholipids and naturally-derived phospholipids extracted from rabbit cerebra, cattle cerebra, human placentas, soybeans, and egg yolk. In view of the reaction properties and quality of the reagent, the phospholipid is preferably a synthesized phospholipid.

The concentration of the phospholipid other than the PG included in the reagent is not particularly limited. The phospholipid concentration is suitably decided depending on the type of the phospholipid and conditions of the APTT measurement. For example, the concentration of the phospholipid other than the PG is preferably 30 to 2000 µg/mL, and more preferably 100 to 600 µg/mL. In the case where three kinds of phospholipids, PE, PC, and PS, are used as the phospholipid other than the PG, the concentration of PE included in the reagent according to the present invention is preferably 10 to 700 µg/mL and more preferably 30 to 300 µg/mL, the concentration of PC is preferably 20 to 1000 µg/mL and more preferably 30 to 500 µg/mL, and the concentration of PS is preferably 3 to 300 µg/mL and more preferably 5 to 150 µg/mL.

The reagent may further contain an additive to improve the shelf life and stability of the reagent. The additive may be any additive conventionally used in the APTT measurement. Examples of the additive are antiseptic agents, antioxidants, and stabilizers. Examples of the antiseptic agents are sodium azide, phenols, and antibiotics conventionally used (for example, ciprofloxacin). An example of the antioxidants is butylhydroxyanisole. The stabilizers are, for example, macromolecule polymers. Particularly preferable examples of the stabilizers are polyethyleneglycol and polyvinylpyrrolidone.

The scope of the present invention includes a reagent kit in which the reagent according to the present invention is used. The reagent kit for APTT measurement according to the present invention (hereinafter, may be simply called "reagent kit") includes a first reagent containing an activator and PG at a concentration equal to or greater than 25 µg/mL, and a second reagent containing a calcium salt.

According to the present embodiment, the activator-containing reagent for APTT measurement according to the present invention is suitably used as the first reagent. Therefore, PG included in the first reagent is similar to the PG included in the reagent according to the present invention described above. More specifically, the PG included in the first reagent may be any one of synthesized PGs and naturally-derived PGs. However, the PG is preferably a synthesized PG. The concentration of the PG included in the first reagent is equal to or greater than 25 µg/mL. The concentration of the PG is suitably selected from a range of values that prevents the first reagent from undergoing cloudiness and/or precipitation. A particularly preferable range of values of the concentration is 50 to 150 µg/mL.

The activator included in the first reagent is similar to the activator used in the reagent according to the present invention. For example, the activator is at least one selected from ellagic acid, kaolin, cerite, colloidal silica, and silicic anhydride. Of these examples, ellagic acid or colloidal silica is preferably used.

According to the present embodiment, a solvent used in the reagent is not particularly limited as far as the PG and the activator can be dissolved therein and the APTT measurement is not thereby disturbed. The solvent is preferably a buffer solution conventionally used in reagents for APTT measurement. Examples of the buffer solution are HEPES, TRIS, and MOPS. The pH of the first reagent is preferably 5 to 9, and more preferably 6 to 8.

According to the present embodiment, the first reagent preferably further contains a phospholipid other than the PG. Similarly to the phospholipid used in the reagent according to the present invention, the phospholipid according to the present embodiment is, for example, at least one selected from PE, PC, and PS. The concentration of the phospholipid other than the PG included in the first reagent is set to a range of values similar to the range of values given in the description of the reagent according to the present invention.

According to the present embodiment, the first reagent may further contain an additive to improve the shelf life and stability of the reagent. The additive to be added for the purpose is similar to the additive used in the reagent according to the present invention.

The calcium salt included in the second reagent may be any one of calcium salts conventionally used in the APTT measurement. The calcium salt is suitably obtained from calcium and one selected from salts of inorganic acids and salts of organic acids. Examples of the calcium salts are calcium chloride, calcium sulfate, calcium nitrite, calcium carbonate, calcium lactate, and calcium tartrate. Of these examples, calcium chloride is preferably used. The concentration of the calcium salt included in the second reagent is suitably decided depending on the type of the calcium salt and conditions of the APTT measurement. The concentration of the calcium salt is preferably 15 to 50 mM, and more preferably 20 to 30 mM. When calcium chloride is used as the calcium salt, the concentration thereof is preferably 15 to 30 mM, and more preferably 20 to 25 mM.

The reagent kit according to the present invention may contain, in addition to the first and second reagents, other reagents conventionally used for APTT measurement, for example, blood plasma for reference. Examples of the blood plasma for reference are normal blood plasma, blood plasma for accuracy control, blood plasmas with coagulation factor deficiencies, LA-positive blood plasma, and blood plasma containing factor VIII inhibitor.

The PG is added to the reagent and the reagent kit described so far in order to lower the sensitivity to LA. On the other hand, the sensitivity of the reagent and reagent kit to coagulation factors under the normal conditions of APTT measurement is substantially equal to the conventional reagents for APTT measurement. A manner of use of the reagent and the reagent kit is not different from that used for the conventional reagents for APTT measurement. Therefore, the reagent and the reagent kit, when used for normal blood plasma under the normal conditions of APTT measurement, can obtain a measurement result similar to measurement results of the conventional reagents for APTT measurement.

The scope of the present invention includes a method for measuring APTT wherein the reagent kit according to the present invention is used. The method for measuring APTT according to the present invention (hereinafter, may be simply called "measuring method") includes a first mixing step. In the first mixing step, blood plasma to be tested is mixed with a first reagent containing an activator and PG at a concentration equal to or greater than 25 µg/mL. This first reagent is similar to the first reagent included in the reagent kit according to the present invention.

The blood plasma to be tested is not particularly limited as far as blood plasma obtained by isolating blood cells from whole blood is used. Examples of the blood plasma to be tested are blood plasma obtained from blood collected from a test subject, the blood plasma for reference, and a mixture of these blood plasmas. A conventional technique can be employed to obtain blood plasma from whole blood. To obtain blood plasma, for example, blood cells are isolated by subjecting blood to centrifugal separation to an extent that hemolysis does not occur. An anticoagulant conventionally used in clinical tests of coagulability may be added to blood collected from a test subject. An example of the anticoagulant is trisodium citrate.

A mixing ratio (volume ratio) of the blood plasma to be tested and the first reagent is preferably 8:2 to 2:8, more preferably 6:4 to 4:6, and even more preferably 5:5. A reaction time of the blood plasma to be tested and the first reagent is preferably one to ten minutes, and more preferably three to five minutes. A preferable range of temperatures to be set is 25 to 45°C, and a more preferable range of temperatures is 35 to 38°C.

In a second mixing step, a sample obtained in the first mixing step is mixed with a second reagent. This second reagent is similar to the second reagent included in the reagent kit according to the present invention. A mixing ratio (volume ratio) of the sample and the second reagent is preferably 8:2 to 5:5, more preferably 7:3 to 6:4, and even more preferably 2:1. A range of temperatures to be set is similar to the range of temperatures given in the description of the first mixing step.

In a step of measuring coagulation time, the coagulation time of the sample obtained in the second mixing step is measured. According to the present embodiment, a person may measure fibrin deposition time through visual observation using a stopwatch or may measure the time using an automatic analyzer. The analyzer may be, for example, a device configured to measure optical information such as changes of transmitted lights and scattered lights obtained from the sample. Suitable examples of the measuring device are commercially available automatic analyzers equipped with an optical information detector, for example, CS-2000i and CS-5000 supplied by Sysmex Corporation.

The scope of the present invention includes a method for determining blood coagulation abnormalities associated with any etiologic factors but LA (i.e. other than LA) in which the method for measuring APTT is used. According to the present invention, the method for determining blood coagulation abnormalities associated with any etiologic factors but LA (hereinafter, may be simply called "determining method") includes a first mixing step. In the first mixing step, blood plasma to be tested is mixed with a first reagent containing an activator and PG at a concentration equal to or greater than 25 µg/mL. The first mixing step is performed similarly to the first mixing step of the method for measuring APTT according to the present invention. The blood plasma to be tested and the first reagent are similar to those given in the description of the measuring method and the reagent kit according to the present invention.

The blood coagulation abnormalities associated with any etiologic factors but LA are not particularly limited. Such blood coagulation abnormalities include all of coagulation abnormalities with prolonged APTT associated with any etiologic factors other than the presence of LA in the blood plasma to be tested. Examples of such abnormalities are disorders associated with coagulation factor deficiencies or abnormalities and coagulation factor inhibitors. Such disorders are specifically, for example, hemophilia A, hemophilia B, Von Willebrand disease, factor XI deficiency, factor XII deficiency, high molecular-weight kininogen deficiency, prekallikrein deficiency, vitamin K deficiency, and acquired hemophilia.

According to another embodiment of the present invention, a cross mixing test is employed to determine the presence or absence of blood coagulation abnormalities associated with any etiologic factors but LA, whereby three different types of blood plasmas are used as the blood plasma to be tested; blood plasma collected from a test subject, normal blood plasma, and a blood plasma in which these blood plasmas are mixed in a predetermined ratio (mixed blood plasma). The mixed blood plasma is at least a blood plasma in which the blood plasma to be tested and normal blood plasma are mixed by the volume ratio of 1:1. Preferably, the mixed blood plasma used are three different blood plasmas in which the blood plasma to be tested and normal blood plasma are mixed by the volume ratios of 8:2, 5:5, and 2:8, respectively. After the blood plasma to be tested and normal blood plasma are mixed, the mixed blood plasma is preferably subjected to incubation. An incubation time is suitably decided depending on the mixing ratio and blood coagulation abnormality to be identified. Taking, for instance, blood plasma suggesting possible presence of a coagulation factor inhibitor such as acquired hemophilia, the incubation time is preferably 0.5 to four hours, and more preferably one to three hours. A range of temperatures during the incubation is preferably 25 to 45°C, and more preferably 35 to 38°C.

The second mixing step and the coagulation time measuring step of the determining method according to the present invention can be performed in a manner similar to the method for measuring APTT according to the present invention, described above. The second reagent is similar to the second reagent included in the reagent kit according to the present invention described earlier.

The determining method according to the present invention determines, based on the coagulation time obtained in the measuring step, whether blood coagulation abnormalities associated with any etiologic factors other than LA are present in the blood plasma to be tested. The sensitivity to LA is weakened in the reagent kit according to the present invention. Therefore, when the APTT measured on the blood plasma to be tested is greater than a predetermined threshold, the blood plasma to be tested may be determined as having a higher likelihood of blood coagulation abnormality associated with an etiologic factor other than LA. The threshold can be decided based on accumulated APTT data of normal blood plasmas collected from healthy subjects. An upper limit of normal is "average value + 1.96 SD (standard deviation)" obtained by statistical calculation of APTT data on blood plasmas collected from more than 100 healthy subjects. The APTT data of normal blood plasmas obtained by the use of the reagent according to the present invention may be accumulated to define an APTT normal range from the data. In that case, any blood plasmas to be tested beyond the normal range may be suspected to have blood coagulation abnormalities associated with any etiologic factors but LA.

When a normal blood plasma, such as Coagtrol N (Sysmex Corporation), is used as the blood plasma to be tested, an APTT ratio may be calculated. In that case, values of the APTT ratio greater than a predetermined threshold, for example, are defined as an indicator for suspecting blood coagulation abnormalities associated with any etiologic factors but LA. The APTT ratio is calculated by dividing an APTT value of blood plasma collected from a test subject by an APTT value of the normal blood plasma. The threshold of the APTT ratio can be decided based on accumulated APTT data of normal blood plasmas collected from healthy subjects. For example, the threshold may be equal to or greater than 1.2.

When employing a cross mixing test to determine the presence or absence of blood coagulation abnormalities associated with any etiologic factors but LA, a line graph may be drawn, in which the horizontal axis represents the mixing ratio of the blood plasma to be tested and the normal blood plasma, and the vertical axis represents APTT. This graph identifies two different blood coagulation abnormalities associated with any etiologic factors but LA. More specifically, in the line graph, a line with a U-like curve indicates a possibility of coagulation factor deficiency or abnormality. On the other hand, a line with a reversed U-like curve in the line graph indicates a possible presence of coagulation factor inhibitor.

According to the present embodiment, the steps of the determining method may be performed by a manual method. Preferably however, all of the steps of the determining method are performed by an automatic analyzer to screen more accurately. Such an analyzing device may be any one of available devices configured to automatically measure APTT and compare measured values to a threshold to display a result thereby obtained. For example, a full automatic blood coagulation measuring device, CS-2000i (Sysmex Corporation), is suitably used.

Hereinafter, examples of the present invention are described in detail. None of these examples, however, necessarily limits the scope of the present invention.

### Examples

### Example 1: We studied how sensitivity to LA was affected by addition of phosphatidylglycerol.

### (1-1) Preparation of reagents for APTT measurement

### A conventional reagent for APTT measurement not containing PG (reagent A) was prepared. Neither cloudiness nor precipitation was noted in the reagent A. Reagent A

The reagent A contains an activator; ellagic acid (KISHIDA CHEMICAL CO., LTD.) at the concentration of 0.1 mmol/L, phospholipids; L-α-phosphatidylethanolamine (Avanti Polar Lipids, Inc.) at the concentration of 140 µg/mL, L-α-phosphatidylcholine (Avanti Polar Lipids, Inc.) at the concentration of 160 µg/mL, and L-α-phosphatidylserine (Avanti Polar Lipids, Inc.) at the concentration of 40 µg/mL, a buffer solution; an HEPES buffer solution (pH7.35) at the concentration of 50 mM, an antiseptic agent; 0.1% sodium azide, and an antioxidant; 3-t-butyl-4-hydroxyanisole (NACALAI TESQUE, INC.).

As the reagent according to the present invention, PG-containing reagents for APTT measurement (reagents B to E) were prepared. Neither cloudiness nor precipitation was noted in any of these reagents.

### Reagents B to E

The reagents B to E were prepared by adding L-α-phosphatidylglycerol (Avanti Polar Lipids, Inc.) to the reagent A at the concentrations of 25, 50, 75, and 100 µg/mL, respectively.

Other than these reagents A to E, a control reagent was used; a commercially available reagent for APTT measurement, Actin SL (Siemens Japan K. K.). This reagent contains a naturally-derived phospholipid extracted from rabbit cerebra and soybeans.

### (1-2) APTT measurement

### (i) Samples

As samples, normal blood plasmas (29 subjects), blood plasmas suspected to contain LA (six subjects), and blood plasmas with coagulation factor deficiencies (eight subjects) were used. Additionally, a normal blood plasma commercially available, Coagtrol N (Sysmex Corporation), was used to calculate Rosner index values.

### (ii) APTT measurement

Each of the samples (50 µL) was dispensed in a cuvette and heated at 37°C for one minute. The reagents for APTT measurement preheated at 37°C (50 µL) were added to these samples and reacted with the samples at 37°C for three minutes. Then, a calcium chloride solution (50 µL) was mixed with the reacted solutions at the concentration of 25 mM to measure coagulation time of each. To obtain an index of sensitivity to LA with each of the reagents, the Rosner index values were calculated in accordance with the method of Rosner. E, et al. (Thromb. Haemast. 1987, 57:144-147). Similarly, for blood plasma containing a mix of LA-suspected blood plasma and the normal blood plasma (Coagtrol N) by the ratio of 1:1 (1:1 mixed blood plasma), coagulation time was measured and calculated by the following formula. To measure the coagulation time, the full automatic coagulation measuring device CS-2000i (Sysmex Corporation) was used. The coagulation time was measured twice for each sample to obtain an average coagulation time.

Rosner index value = [((APTT of 1:1 mixed blood plasma) - (APTT of normal blood plasma))/(APTT of LA-suspected blood plasma)] × 100 ... formula (1).

Table 1 shows an obtained result with phospholipid compositions of the respective samples. Referring to Table 1, "LA-positive blood plasma" is blood plasma determined as containing LA among the six blood plasmas suspected to contain LA. Referring to the table, "Test (second)" shows coagulation time of LA-positive blood plasma, and "1:1 mixing (second)" shows coagulation time of blood plasma mixedly containing LA-positive blood plasma and normal plasma by the ratio of 1:1.

**Table 1**

| Phospholipid (concentration) | | | Reagent A | Reagent B | Reagent C | Reagent D | Reagent E | | Commercial products FSL |
|---|---|---|---|---|---|---|---|---|---|
| Composition | PE (µg/mL) | | 140 | 140 | 140 | 140 | 140 | | |
| | PC (µm/mL) | | 160 | 160 | 160 | 160 | 160 | | <Derived from> Rabbits Soybeans |
| | PS (µg/mL) | | 40 | 40 | 40 | 40 | 40 | | |
| | PG (µg/mL) | | 0 | 25 | 50 | 75 | 100 | | |
| | | | | | | | | | |
| | Coagulation time of normal blood plasma (sec.) | | 27.0 | 27.8 | 28.5 | 29.3 | 30.0 | | 28.8 |
| | | | | | | | | | |
| | LA-positive blood plasma | Test (sec.) | 52.3 | 48.5 | 46.3 | 43.5 | 36.2 | | 63.5 |
| | | 1:1 mixing (sec.) | 36.8 | 34.1 | 31.7 | 30.6 | 29.3 | | 44.7 |
| | | Rosner Index | 23.3 | 18.9 | 13.8 | 11.4 | 9.1 | | 27.4 |

### (1-3) Performance evaluation

Referring to Table 1, the normal plasma coagulation time resulted in 27 to 30 seconds with all of the reagents. In the relevant technical field, APTT of normal blood plasma within the range of 26.0 to 30.0 seconds is a level of performance conventionally required for reagents for APTT measurement. This result confirmed that the addition of PG to the reagents for APTT measurement did not affect the measurement result of the normal blood plasmas.

Table 1 teaches that the APTT and Rosner index values of the LA-containing blood plasmas lowered depending on the PG concentrations of the respective reagents. The reagent for APTT measurement according to the present invention was proven to have a lowered sensitivity to LA. On the other hand, the reagent A and the commercial products not containing PG had large Rosner index values, presenting high sensitivities to LA. A measurement result of blood plasmas with coagulation factor deficiencies (no data illustrated in this specification) teaches that the addition of PG does not affect the sensitivity to coagulation factors.

### Example 2: We studied how sensitivity to LA was affected by adjustments of phospholipid compositions.

### (2-1) Preparation of reagents for APTT measurement

Reagents for APTT measurement not containing PG but respectively containing PE, PC, and PS at different concentrations (reagents F to N) were prepared. All of the reagents contain an activator; ellagic acid (KISHIDA CHEMICAL CO., LTD.) at the concentration of 0.1 mmol/L, a buffer solution; an HEPES buffer solution (pH 7.35) at the concentration of 50 mM, an antiseptic agent; 0.1% sodium azide, and an antioxidant; 3-t-butyl-4-hydroxyanisole (NACALAI TESQUE, INC.). The phospholipid compositions of the respective reagents are illustrated below.

### Reagents F to J

All of the reagents F to J contain L-α-phosphatidylethanolamine (Avanti Polar Lipids, Inc.) at the concentration of 140 µg/mL and L-α-phosphatidylcholine (Avanti Polar Lipids, Inc.) at the concentration of 160 µg/mL. These reagents, however, respectively contain L-α-phosphatidylserine (Avanti Polar Lipids, Inc.) at different concentrations. Specifically, the reagents F to J respectively contain L-α-phosphatidylserine at the concentrations of 10, 20, 30, 40, and 50 µg/mL. The phospholipid composition of the reagent I was a standard composition of reagents for APTT measurement.

### Reagents K and L

The reagents K and L both contain L-α-phosphatidylcholine (Avanti Polar Lipids, Inc.) at the concentration of 160 µg/mL and L-α-phosphatidylserine (Avanti Polar Lipids, Inc.) at the concentration of 40 µg/mL. However, the reagents K and L respectively contain L-α-phosphatidylcholine (Avanti Polar Lipids, Inc.) at different concentrations. Specifically, the reagents K and L respectively contain L-α-phosphatidylcholine at the concentrations of 40 µg/mL and 250 µg/mL.

### Reagents M and N

The reagents M and N both contain L-α-phosphatidylethanolamine (Avanti Polar Lipids, Inc.) at the concentration of 140 µg/mL and L-α-phosphatidylserine (Avanti Polar Lipids, Inc.) at the concentration of 40 µg/mL. However, the reagents M and N respectively contain L-α-phosphatidylcholine (Avanti Polar Lipids, Inc.) at different concentrations. Specifically, the reagents M and N respectively contain L-α-phosphatidylcholine at the concentrations of 40 µg/mL and 320 µg/mL.

Other than these reagents F to N, the reagent for APTT measurement according to the present invention, the reagent E, was used as a control reagent.

### (2-2) APTT measurement

This example used the same samples as the example 1. As to these samples, coagulation time was measured by using the reagents prepared in 2-1 in a manner similar to the example 1 to calculate Rosner index values. Table 2 shows an obtained result with phospholipid compositions of the respective reagents.

**Table 2**

| Phospholipid (concentration) | | | Reagent F | Reagent G | Reagent H | Reagent I | Reagent J | | Reagent K | Reagent L | | Reagent M | Reagent N | | Present invention |
|---|---|---|---|---|---|---|---|---|---|---|---|---|---|---|---|
| Composition | PE (µg/mL) | | 140 | 140 | 140 | 140 | 140 | | 40 | 250 | | 140 | 140 | | 140 |
| | PC (µg/mL) | | 160 | 160 | 160 | 160 | 160 | | 160 | 160 | | 40 | 320 | | 160 |
| | PS (µg/mL) | | 10 | 20 | 30 | 40 | 50 | | 40 | 40 | | 40 | 40 | | 40 |
| | PG (µg/mL) | | 0 | 0 | 0 | 0 | 0 | | 0 | 0 | | 0 | 0 | | 100 |
| | | | | | | | | | | | | | | | |
| | Coagulation time of normal blood plasma (sec.) | | 31.5 | 27.8 | 29.4 | 26.3 | 25.4 | | 25.0 | 28.1 | | 25.9 | 30.6 | | 27.4 |
| | | | | | | | | | | | | | | | |
| | LA-positive blood plasma | Test (sec.) | 58.3 | 46.3 | 44.1 | 42.9 | 41.0 | | 40.3 | 47.9 | | 43.2 | 44.9 | | 38.7 |
| | | 1:1 mixing (sec.) | 46.8 | 37.0 | 34.2 | 33.6 | 31.7 | | 31.2 | 36.3 | | 33.4 | 36.8 | | 30.2 |
| | | Rosner Index | 22.8 | 14.3 | 12.9 | 11.7 | 11.0 | | 9.9 | 11.7 | | 12.3 | 11.1 | | 7.8 |

### (2-3) Performance evaluation

Reviewing the result obtained from the reagents F to J, the APTT and Rosner index values of the LA-containing blood plasmas showed reductions with higher PS concentration values. However, the values obtained from these reagents were still greater than the value obtained from the reagent according to the present invention. With the reagents F and J, the APPT of the normal blood plasmas was beyond the range of 26 to 30 seconds. Further, there was no measurable difference among the Rosner index values obtained from the reagents having the PS concentrations of 30 to 50 µg/mL. Though no data is illustrated, the occurrence of cloudiness and/or precipitation was noted in the reagents of this example containing PS at the concentrations greater than 50 µg/mL because PS could not be completely dissolved in these reagents. This demonstrates a difficulty in lowering the sensitivity to LA through adjustments of the PS concentration.

Reviewing the result obtained from the reagents I, K, and L, the APTT and Rosner index values of the LA-containing blood plasmas showed reductions with lower PE concentration values. However, the values obtained from these reagents were still greater than the value obtained from the reagent according to the present invention. With the reagent K, the APPT of the normal blood plasmas was beyond the range of 26 to 30 seconds. This demonstrates a difficulty in lowering the sensitivity to LA through adjustments of the PE concentration. Though no data is illustrated, the occurrence of cloudiness and/or precipitation was noted in the reagents of this example containing PE at the concentrations greater than 250 µg/mL because PE could not be completely dissolved in these reagents.

Reviewing the result obtained from the reagents I, M, and N, the APTT and Rosner index values of the LA-containing blood plasmas showed reductions with higher PC concentration values. However, the values obtained from these reagents were still greater than the value obtained from the reagent according to the present invention. With the reagents M and N, the APPT of the normal blood plasmas was beyond the range of 26 to 30 seconds. Though no data is illustrated, the occurrence of cloudiness and/or precipitation was noted in the reagents of this example containing PC at the concentrations greater than 320 µg/mL because PC could not be completely dissolved in these reagents. This demonstrates a difficulty in lowering the sensitivity to LA through adjustments of the PC concentration.

## Claims

1. A method for measuring activated partial thromboplastin time, comprising steps of:
first mixing a blood plasma with a first reagent, wherein the first reagent comprises an activator and phosphatidylglycerol at a concentration equal to or greater than 25 µg/mL;
second mixing a sample obtained in the first mixing step with a second reagent comprising a calcium salt; and
measuring coagulation time of the sample obtained in the second mixing step.

2. The method according to claim 1, wherein the concentration of the phosphatidylglycerol in the first reagent is equal to or smaller than 150 µg/mL.

3. The method according to claim 1 or 2, wherein the concentration of the phosphatidylglycerol in the first reagent is equal to or greater than 50 µg/mL.

4. The method according to any of claims 1 to 3, wherein the phosphatidylglycerol is a synthesized phospholipid.

5. The method according to any of claims 1 to 4, wherein the activator is at least one selected from the group consisting of ellagic acid, kaolin, cerite, colloidal silica, and silicic anhydride.

6. The method according to any of claims 1 to 5, wherein the first reagent further comprises a phospholipid other than the phosphatidylglycerol.

7. The reagent according to claim 6, wherein the phospholipid other than the phosphatidylglycerol is at least one selected from the group consisting of phosphatidylserine, phosphatidylethanolamine, and phosphatidylcholine.

8. A reagent kit for measuring activated partial thromboplastin time, comprising:
a first reagent comprising phosphatidylglycerol; and
a second reagent comprising a calcium salt,
wherein a concentration of the phosphatidylglycerol in the first reagent is equal to or greater than 25 µg/mL.

9. The reagent kit according to claim 8, wherein the concentration of the phosphatidylglycerol in the first reagent is equal to or smaller than 150 µg/mL.

10. The reagent kit according to claim 8 or 9, wherein the concentration of the phosphatidylglycerol in the first reagent is equal to or greater than 50 µg/mL.

11. The reagent kit according to any of claims 8 to 10, wherein the concentration of the phosphatidylglycerol in the first reagent is 50 to 150 µg/mL.

12. Use of the reagent kit according to any of claims 8 to 11, for a method for determining blood coagulation abnormality, the method comprising:
first mixing a blood plasma with a first reagent, wherein the first reagent comprises an activator and phosphatidylglycerol at a concentration equal to or greater than 25 µg/mL;
second mixing a sample obtained in the first mixing step with a second reagent comprising a calcium salt;
measuring coagulation time of the sample obtained in the second mixing step; and
determining presence or absence of a blood coagulation abnormality which is not caused by a lupus anticoagulant in the blood plasma based on the coagulation time obtained in the measuring step.

13. The use according to claim 12, wherein the concentration of the phosphatidylglycerol in the first reagent is 50 to 150 µg/mL.

14. The use according to claim 12 or 13, wherein the blood coagulation abnormality is a disorder associated with coagulation factor deficiency, a disorder associated with coagulation factor abnormality, or a disorder associated with coagulation factor inhibitor.

15. The use according to any of claims 12 to 14, wherein, in the determining step, the blood sample is determined to be suspected to have the blood coagulation abnormality when the coagulation time obtained in the measuring step is greater than a predetermined threshold.
